# EUROPEAN PATENT SPECIFICATION

(11) **EP 3 048 964 B1**
(45) Date of publication and mention of the grant of the patent: **10.04.2019**
(21) Application number: 14790391.8
(22) Date of filing: 24.09.2014
(51) Int. Cl.: G16H 40/63, A61B 5/0408, A61B 5/046, A61B 5/0464, A61B 5/0472, A61B 5/0402, A61B 5/044, A61B 5/0432, A61B 5/0452, A61B 5/04, A61B 5/00, G06F 19/00

(54) **INTERACTIVE PROCESSING OF ECG DATA**
INTERAKTIVE VERARBEITUNG VON EKG-DATEN
TRAITEMENT INTERACTIF DE DONNÉES ECG

(30) Priority: 25.09.2013 US 201361882403 P; 14.11.2013 US 201314080725; 14.11.2013 US 201314080717; 15.11.2013 US 201314082071; 25.07.2014 US 201414341698
(43) Date of publication of application: 03.08.2016
(73) Proprietor: Bardy Diagnostics, Inc., Seattle, WA 98104 (US)
(72) Inventor: DREISBACH, Ezra M., Vashon, Washington 98070 (US); FELIX, Jason, Vashon Island, Washington 98070 (US); BARDY, Gust H., Carnation, Washington 98014 (US)
(74) Representative: Hanna Moore + Curley
(86) International application number: PCT/US2014/057293
(87) International publication number: WO 2015/048182

(56) References cited:
- EP-A2- 1 859 833
- WO-A1-98/52463
- US-A1- 2005 151 640

## Description

### TECHNICAL FIELD

This application relates in general to electrocardiography and, in particular, to a system and method for interactive processing of electrocardiogram (ECG) data.

### BACKGROUND ART

An ECG procedure measures cardiac electrical potentials that can be graphed to visually depict the electrical activity of the heart over time. Conventionally, a standardized set format 12-lead configuration is used by an ECG machine to record cardiac electrical signals from well-established traditional chest locations. Sensed cardiac electrical activity is represented by PQRSTU waveforms that can be interpreted post-ECG recordation to derive heart rate and physiology and for use in medical diagnosis and treatment.

Within an ECG waveform, the P-wave represents atrial electrical activity. The QRSTU components represent ventricular electrical activity. Some cardiac conditions have frequency-specific content. The QRS complex in ventricular tachyarrhythmia, for instance, has a maximum amplitude at 4 Hz, while the frequencies associated with ventricular fibrillation are concentrated in the 4-7 Hz range. Cardiac vagal activity and respiratory sinus arrhythmias are seen in the 0.15-0.50 Hz range. Other frequencies may reflect other cardiac conditions.

Noise in recorded signals or other artifacts that do not reflect cardiac activity can contribute to an incorrect diagnosis of a patient. The main sources of noise in an ECG machine are common mode noise, such as 60Hz power line noise, baseline wander, muscle noise, and radio frequency noise from equipment including pacemakers or other implanted medical devices. Such noise can contribute to an incorrect diagnosis of the patient. For example, electrical or mechanical artifacts, such as produced by poor electrode contact or tremors, can simulate life-threatening arrhythmias. Similarly, baseline wander produced by excessive body motion during an ECG procedure may simulate an ST segment shift ordinarily seen in myocardial ischemia or injury.

Current ECG over-reading software generally does not allow a user to apply an arbitrary noise filter of choice to an ECG trace; users are generally limited to a set of proprietary filters. In addition, conventional over-reading software generally fails to provide users with a way to compare the results of combinations of arbitrary noise filters, thus preventing the user from finding the most appropriate filter. This is especially relevant when trying to record the P-wave or cardiac atrial signal. EP1859833 represents a prior art ECG system.

Therefore, a need remains for a way to facilitate real-time, interactive processing of an ECG.

### DISCLOSURE OF THE INVENTION

The present application provides a system and method for interactive processing of ECG data in accordance with the claims which follow.

Still other embodiments of the present invention will become readily apparent to those skilled in the art from the following detailed description, wherein are described embodiments by way of illustrating the best mode contemplated for carrying out the invention. As will be realized, the invention is capable of other and different embodiments and its several details are capable of modifications in various obvious respects, all without departing from the the scope of the present invention. Accordingly, the drawings and detailed description are to be regarded as illustrative in nature and not as restrictive.

### DESCRIPTION OF THE DRAWINGS

FIGURE 1 is a graph showing, by way of example, a normal ECG waveform for a single cardiac cycle.
FIGURE 2 is a graph showing, by way of example, an ECG waveform of a patient with atrial flutter for a single cardiac cycle, where the ECG waveform has been corrupted by power line noise.
FIGURE 3 is a diagram showing a screen shot generated by an application for interactive processing of ECG data in accordance with one embodiment.
FIGURE 4 is a functional block diagram showing a system for interactive processing of ECG data in accordance with one exemplary configuration.
FIGURE 5 is a flow diagram showing a method for interactive processing of ECG data in accordance with one embodiment.
FIGURE 6 is a flow diagram showing a routine for recommending an ECG filter for use in the method of FIGURE 5 in accordance with one embodiment.

### BEST MODE FOR CARRYING OUT THE INVENTION

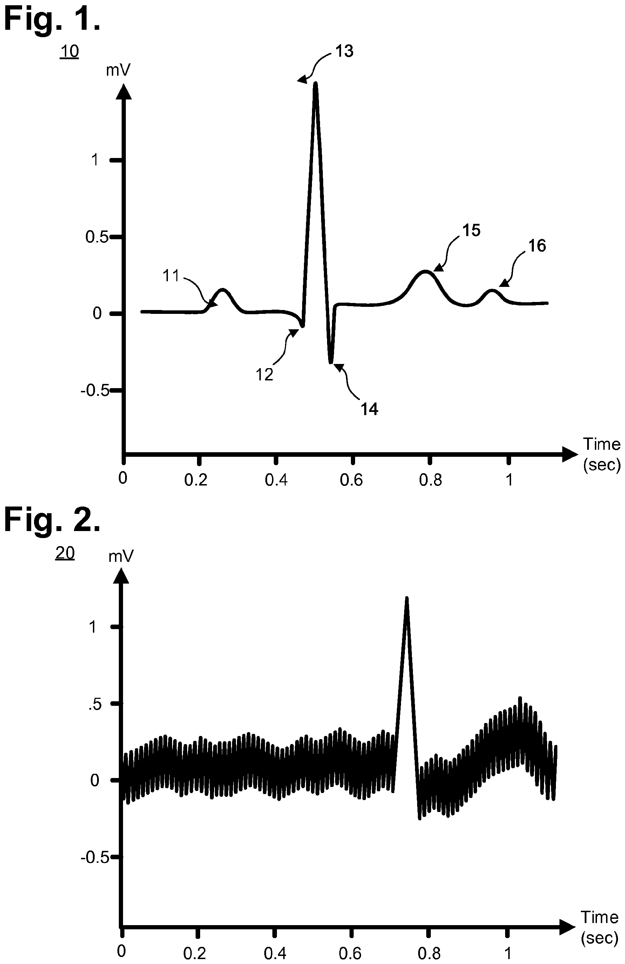

An ECG includes multiple waveforms reflecting multiple contractions of a patient's heart. FIGURE 1 is a graph showing, by way of example, a normal ECG waveform 10 for a single cardiac cycle. The x-axis represents time in approximate units of tenths of a second. The *y*-axis represents cutaneous electrical signal strength in approximate units of millivolts. The P-wave 11, when recorded from the anterior thorax, normally has a smooth, initially upward slope, (i.e., a positive vector) that indicates atrial depolarization from right to left atrium. The QRS complex usually begins with the downward deflection of a Q wave 12, followed by a larger upward deflection of an R-wave 13, and terminated with a downward waveform of the S wave 14, collectively representative of ventricular depolarization. The T wave 15 is normally a modest upward waveform, representative of ventricular repolarization, while the U wave 16, often not directly observable, indicates repolarization of the Purkinje conduction fibers.

ECG signals include low amplitude voltages in the presence of high offsets and noise, which requires the signals to be amplified and filtered prior to being displayed for interpretation. In an unfiltered ECG, some of the features may not be apparent, particularly if their shapes have were received with the ECG 32, the signals corresponding to the selection 34 can be identified among the received signals. If no digitized ECG signals have been received, the application 30 can reconstruct the digitized signals from the selection 34. Other ways to obtain the digitized signals are possible.

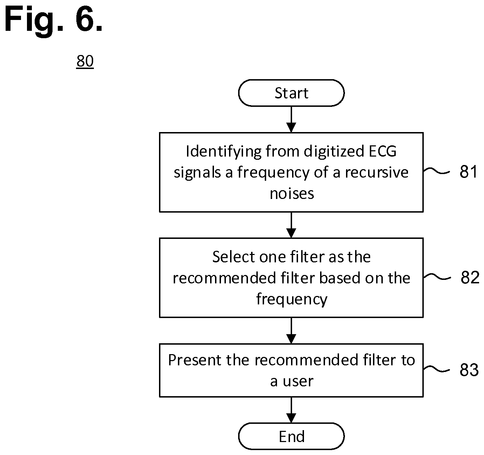

Optionally, the selection is zoomed and the zoomed selection 35 is displayed to the user by the application (step 66). A list, such as in the selection menus 36, 38, of a plurality of digital ECG filters for filtering the selection is displayed to the user, with the user being able to select one or more sets of the filters for filtering the selection (step 67). Optionally, a filter recommended for processing the selection 34 is determined and displayed to the user, as further described with reference to FIGURE 6 (step 68). A user selection of one or more sets of the filters is received by the application 30, with each of the filter sets including at least one of the filters displayed (step 69). The application 30 applies each of the sets of the selected filters to the digitized ECG signals for the selection (step 70), generates filtered ECG for the selection based on the digital signals filtered by each of the sets, and displays the filtered ECG for the selection on portions 37, 39 of the display screen of the user device 31 (step 71). The filtered ECGs can be displayed visually proximate to each other, allowing comparison of results of filtering side-by-side, and thus enabling the user to decide which of the results is more useful, whether one of the results satisfies the user's needs, or whether a still different set of filters needs to be applied. Optionally, upon receiving a user selection of one of the filtered ECGs for the selection, the application 30 can replace the selected portion 34 of the ECG 32 with the selected filtered ECG (step 72), ending the method 60.

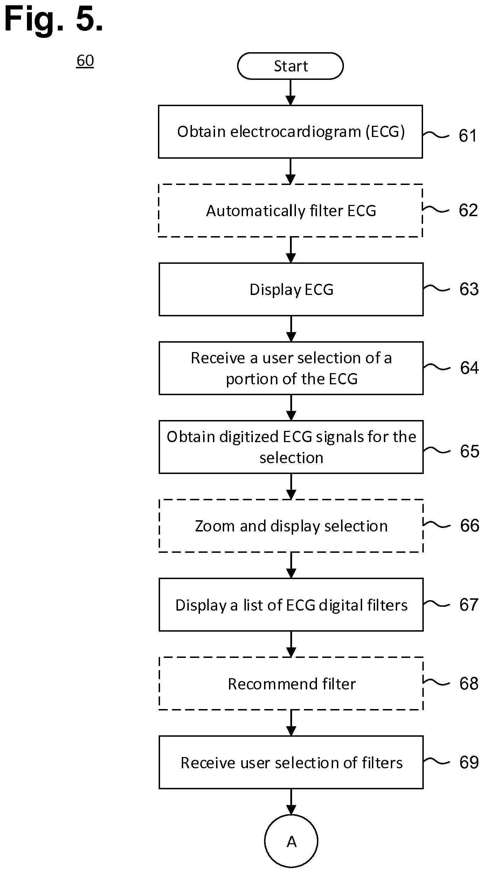

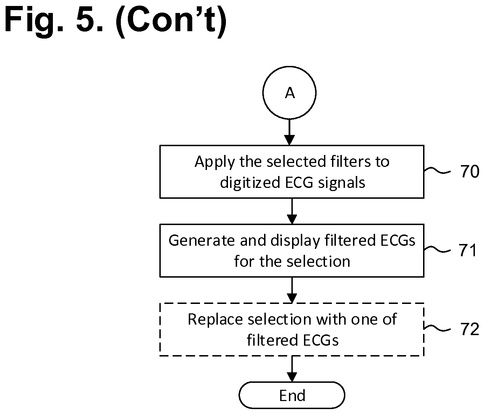

Recommending an ECG filter to the user can save the user time and simplify ECG interpretation for the user. FIGURE 6 is a flow diagram showing a routine 80 for recommending an ECG filter to a user for use in the method 60 of FIGURE 5 in accordance with one embodiment. First, a frequency of a recursive noise present in the ECG selection is identified (step 81). Second, one or more digital filters are selected based on the noise frequency (step 82). For example, if the selection includes high-frequency recursive noise, a low-pass filter can be chosen for the recommendation. Lastly, the selected filter is recommended to a user, terminating the routine 80 (step 83).

While the invention has been particularly shown and described as referenced to the embodiments thereof, those skilled in the art will understand that the foregoing and other changes in form and detail may be made therein without departing from the scope. to clarify each individual ECG and provide for patient-specific ECG signal processing. In addition, digital filters are immune to the effects of wear and degradation that all hardware experiences.

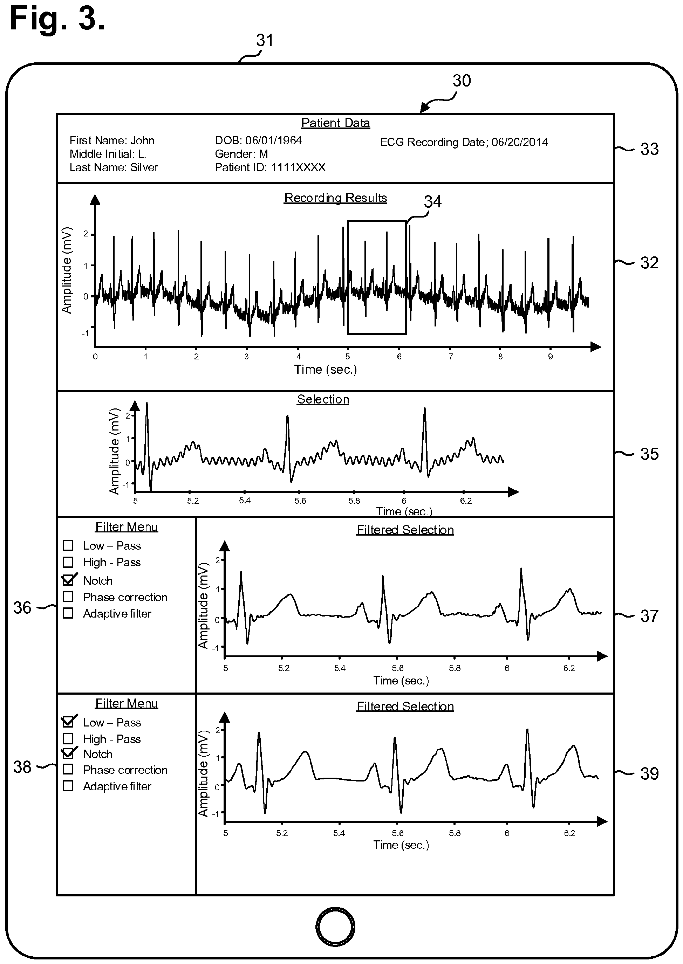

ECG noise can be effectively reduced by allowing a user to pick particular portions of an ECG for application of a filter and allowing the user to compare results of applications of different filters to the selected portions. This is critical when seeking to record the more difficult-to-see P-wave compared to the high voltage high frequency content of the QRS wave. FIGURE 3 is a diagram showing a screen shot generated by an application 30 for interactive processing of ECG data in accordance with one exemplary configuration. The application 30 can be a downloadable application executed on a user device 31. While the user device 31 is shown as a tablet computer with reference to FIGURE 1, other kinds of user devices 31, such as mobile phones, desktop computer, laptop computers, portable media players are possible; still other types of user devices 31 are possible. The user device 31 can include components conventionally found in general purpose programmable computing devices, such as a central processing unit, memory, input/output ports, network interfaces, and non-volatile storage, although other components are possible. The central processing unit can implement computer-executable code, including digital ECG filters, which can be implemented as modules. The modules can be implemented as a computer program or procedure written as source code in a conventional programming language and presented for execution by the central processing unit as object or byte code. Alternatively, the modules could also be implemented in hardware, either as integrated circuitry or burned into read-only memory components. The various implementations of the source code and object and byte codes can be held on a computer-readable storage medium, such as a floppy disk, hard drive, digital video disk (DVD), random access memory (RAM), read-only memory (ROM) and similar storage mediums. Other types of modules and module functions are possible, as well as other physical hardware components.

The application 30 receives results of an ECG monitoring, which can include an ECG 32, including in a printed form. The ECG 32 can be received at once, such as upon completion of monitoring, or in portions, as the monitoring progresses. In addition to the ECG 32, the application 30 can display information about the patient 33 such as the patient's name, date of birth, gender, and patient ID; other clinical or physiological information associated with the patient can also be displayed.

A user may select a portion 34 of the displayed ECG 32 for application of one or more digital filters, such as by clicking on the portion or highlighting the portion with a mouse. The selected portion 34 can be zoomed and displayed in a separate area 35 of the application screen. By looking at the selection in the area 35, the user can decide what filters to apply to the selection 34.

Application of filters to an ECG can result in a loss of clinical information present in the ECG waves. Only a limited number of filters can be applied before such clinical information is lost due to the filters introducing distortions into some part of the ECG signals. For example, a high-pass filter, a filter whose purpose is to remove low-frequency noise, introduces distortions to the ST segment of ECG. The distortion arises from the combination of the frequencies of some of the noise overlapping with the spectra of useful ECG waves, with the noise generally being stochastic; thus any attempt of removing the noises after signal acquisition is typically accompanied by some degree of signal degradation. An excessive number or an incorrect set of applied filters can remove useful diagnostic features from the ECG waveform, leading to false diagnostic statements. By selecting a portion 34 of the ECG and, applying filters only to that portion, the rest of the ECG 32 is maintained intact and unfiltered.

The user may filter the selection 34 using a list of ECG digital noise filters provided by application in filter selection menus 36, 38. By selecting the filters in different menus 36, 38, the user can select different sets of filters for filtering the ECG 32. Each of the digital filters is a mathematical algorithm that is applied to digital ECG signals to output a set of filtered signals that differs from the set of the ECG signals to which that filter is initially applied. The filters can be stored in the memory of the user device 31. Such filters can include a low-pass filter, which attenuates noise with a frequency higher than a cut-off frequency; a high-pass filter, which attenuates signals with frequencies lower than the cut-off frequency; a notch filter, which passes all frequencies except those in a stop-band centered on a center frequency; a phase correction filter, which corrects a phase of an ECG wave following earlier digital processing; and an adaptive filter, which obtains the frequency of the noise present, such as based on patient input or by calculating the noise, and minimizes the identified noise. Other types of filters are possible.

The user can customize the filter selection menus 36, 38. For instance, the user can change the order in which the filters are displayed in the selection menus 36, 38, such as by dragging and dropping the filters with a mouse. Thus, if the user uses particular filters more often than other filters, the more used filters can be brought to the top of the menus 36, 38. Further, the order of the filters in the filter selection menu 36 can be different from the order in the menu 38.

Also, the user can select the displayed filters, such as by clicking on a name of one of the filters, and change one or more parameters of the selected filter. For example, if the selected filter is a high-pass filter, the user can enter a cut-off frequency used for the filter. Other parameters can also be changed. The desired parameters can be changed in a separate window of the application 32 that appears upon the filter being selected, though other ways for the user to change the parameters are possible. Still other ways to customize the filter selection menus are possible.

The user may apply different filters or combinations of filters to the selection 34, and see the results of applications of different filters side-by-side in the areas 37 and 39. For example, the user may select a notch filter to be applied to the selection 34, and see the results of the application of the filter, a filtered ECG of the selection, in the area 37. While the application of the notch filter results in a clearer shape of the selection 34, including that of the P-wave, if the user is still not satisfied with the result, the user can choose in the filter selection menu 38 to choose to apply a different set of filters, choosing the notch filter in combination with the low-pass filter to further remove the noise from the selection 34, with the results of the application of the filters being displayed in the area 39. The user can compare the application of different selected filters side-by-side and decide whether any of the applied filters or combinations of filters produce a satisfactory result or whether applications of other filters are necessary. The results of application of different filters to the selection 34 are displayed to the user immediately upon becoming available, allowing the user to explore different filter set possibilities in real-time and reducing the time necessary to find the most appropriate filter set.

If the user is satisfied with a filtered ECG of the selection in the area 37 or 39, the user can replace the selection 34 of the ECG 32 with the filtered ECG of the selection in area 37 or 39, such as by dragging the selection in the area 37, 39 to the displayed ECG 32 or pressing a button on the screen of the application 31 (not shown).

While two sets of filter selection menus 36, 38 and areas with the results of filter application 38, 39 are shown in the screen of the application, in a further embodiment, other numbers of filter menus and areas showing results of the filtering using the selected filters are possible.

As further described with reference to FIGURE 5 and 6, the application 30 can make a recommendation (not shown) of one or more filters to be applied to the selection 34. The recommendation is created by identifying a frequency of a noise recurring in the selection 34 ("recursive noise"), such as presence of 60 Hz power line noise, based on one or more of user input or mathematical estimation of the noise frequency, and recommending the frequency based on the noise. For example, if the recursive noise includes power line noise, a notch filter or a low-pass filter can be recommended to remove the noise. The recommendation can be presented in different ways, such as presenting the recommendation in a separate field on the screen of the application 30 or by highlighting the filters presented in the menus 36, 38.

In a further embodiment, in addition to providing a filtering recommendation, the application 30 can automatically apply one or more filters to an ECG prior to presenting the ECG to the user, saving the user the labor of filtering noise that can be automatically identified and removed. The application 30 can identify the presence of noise in an ECG received from an ECG monitor or from another source, automatically apply a filter or a combination of filters to digitized signals for portions of the ECG with the noise, and generate the ECG 32 that is displayed to the user based on digitized signals that have been filtered and any digitized signals that did not include the noise. For example, if the application 30 identifies baseline wander corrupting a received ECG, which can be identified using techniques such as measuring deviation of signals from the baseline in a random fashion within set frequency domains, the application 30 can automatically apply a filter or a set of filters to digitized signals for portions of the ECG with the baseline wander, and generate the ECG 32 displayed to the user based on digitized signals that have been filtered and signals that have not been corrupted by the baseline wander. The filters to be applied can be determined via testing, such by as applying different filters, such as various high-pass filters, or combinations of filters to the digitized signals and identifying the filters or combinations of filters that result in the greatest reduction of the baseline wander. In a further embodiment, a preset filter or combination of filters can be used to automatically reduce or remove the baseline wander. In a still further embodiment, the application 30 can also test effect of changing parameters of the filters on the removal of the noise, and choose the most appropriate parameters for the filters used. Other kinds of automated application of filters are possible.

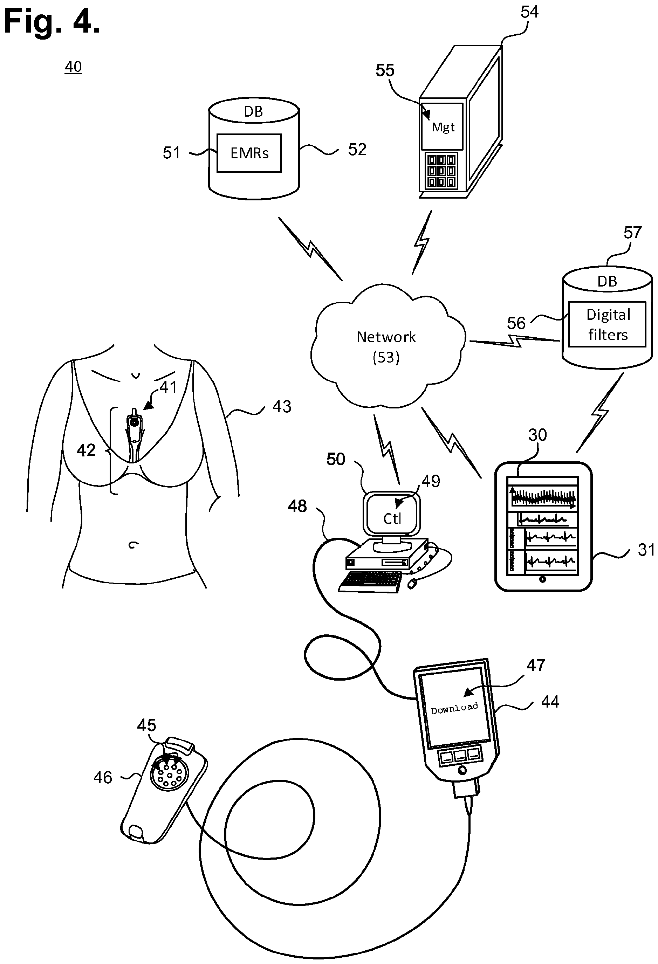

The application can obtain results of an ECG recording from a variety of sources. FIGURE 4 is a functional block diagram showing a system 40 for interactive processing of ECG data in accordance with one embodiment. As seen in FIGURE 2, the application 30 can receive the results from a long-term ECG monitor, a monitor that continuously monitors patient information over a number of days.

In one embodiment, the long-term electrocardiography ECG monitor can be the extended wear ambulatory physiological sensor monitor 41 described in detail in a commonly-assigned U.S. Patent application, entitled "Extended Wear Ambulatory Electrocardiography and Physiological Sensor Monitor," Serial No. 14/080,725, filed November 14, 2013, pending, the disclosure of which is incorporated by reference. The placement of the wearable monitor 41 in a location at the sternal midline 42 (or immediately to either side of the sternum) of the patient 43 significantly improves the ability of the wearable monitor 41 to cutaneously sense cardiac electric signals, particularly the P-wave (or atrial activity) and, to a lesser extent, the QRS interval signals in the ECG waveforms that indicate ventricular activity, while simultaneously facilitating comfortable long-term wear for many weeks. As further described in detail in commonly-assigned U.S. Patent application, entitled "Remote Interfacing of Extended Wear Electrocardiography and Physiological Sensor Monitor," Serial No. 14/082,071, filed on November 15, 2013, pending, the disclosure of which is incorporated by reference, upon completion of the monitoring period, the monitor 41 can be connected to a download station 44, which could be a programmer or other device that permits the retrieval of stored ECG monitoring data, execution of diagnostics on or programming of the monitor recorder 41, or performance of other functions. The monitor 41 has a set of electrical contacts (not shown) that enable the monitor recorder 41 to physically interface to a set of terminals 45 on a paired receptacle 46 of the download station 44. In turn, the download station 44 can execute a communications or offload program 47 ("Offload") or similar program that interacts with the monitor recorder 41 via the physical interface to retrieve the stored ECG monitoring data. The download station 44 could be the user device 31 or another server, personal computer, tablet or handheld computer, smart mobile device, or purpose-built programmer designed specific to the task of interfacing with a monitor 41. Still other forms of download station 44 are possible.

Upon retrieving stored ECG monitoring data from the monitor 41, middleware (not shown) first operates on the retrieved data to adjust the ECG capture quality, as necessary, and to convert the retrieved data into a format suitable for use by third party post-monitoring processing software, such as the application 30. If the download station 44 is not the user device 31, the formatted data can then be retrieved from the download station 44 over a hard link 48 using a control program 49 ("Ctl") or analogous application executing on a personal computer 50 or other connectable computing device, via a communications link (not shown), whether wired or wireless, or by physical transfer of storage media (not shown). The personal computer 50 or other connectable device may also execute middleware that converts ECG data and other information into a format suitable for use by a third-party post-monitoring processing program, such the application 30. Note that formatted data stored on the personal computer 50 would have to be maintained and safeguarded in the same manner as electronic medical records (EMRs) 51 in a secure database 52, as further discussed *infra.* In a further embodiment, the download station 44 is able to directly interface with other devices over a computer communications network 53, which could be some combination of a local area network and a wide area network, including the Internet, over a wired or wireless connection. Still other forms of download station 44 are possible. In addition, the wearable monitor 41 can interoperate with other devices, as further described in detail in commonly-assigned U.S. Patent application, entitled "Remote Interfacing of Extended Wear Electrocardiography and Physiological Sensor Monitor," Serial No. 14/082,071, filed on November 15, 2013, pending. In addition, the wearable monitor 41 is capable of interoperating wirelessly with mobile devices, including so-called "smartphones," such as described in in commonly-assigned U.S. Patent application, entitled "Computer-Implemented System And Method for Providing A Personal Mobile Device-Triggered Medical Intervention," Serial No. 14/217,402, filed on March 17, 2014.

Other kinds of long-term monitors, such as Holter monitors (not shown), can be used to obtain the data processed by the application 30. In addition, the application 30 can receive the results from other kinds of ECG monitors, such as a standard 12-lead ECG monitor (not shown) that records a patient's ECG during a visit to a doctor's office, which can provide the results to the application 30 through the download station 44 or in other ways described above. Still other kinds of ECG and physiological monitors, from which data can be received, are possible. Further, in one embodiment, the results can be obtained by the application 30 upon the completion of the monitoring. In a further embodiment, the results can be provided to the application 30 running on the user device 31 as they are obtained.

While as mentioned above the user device 31 can be the download station 44 and receive ECG recording data from an ECG monitor directly, the application 30 running on the user device 31 can also receive results of monitoring from other sources, such as from a server 54 storing results of completed recordings or "monitorings". A client-server model could be used to employ a server 54 to remotely interface with the download station 44 over the network 53 and retrieve the formatted data or other information. The server 54 executes a patient management program 55 ("Mgt") or similar application that stores the retrieved formatted data and other information in the secure database 52 cataloged in that patient's EMRs 51. The application 30 can receive the results of the monitoring from the server 54. In addition, the patient management program 55 could manage a subscription service that authorizes a monitor recorder 41 to operate for a set period of time or under pre-defined operational parameters.

The patient management program 55, or other trusted application, also maintains and safeguards the secure database 52 to limit access to patient EMRs 51 to only authorized parties for appropriate medical or other uses, such as mandated by state or federal law, such as under the Health Insurance Portability and Accountability Act (HIPAA) or per the European Union's Data Protection Directive. For example, a physician may seek to review and evaluate his patient's ECG monitoring data, as securely stored in the secure database 52.

Still other sources from which the application 30 can receive the results of the ECG monitoring are possible.

As mentioned above, the application 30 applies one or more of ECG digital filters to a user selection of a displayed ECG trace 32. The application 30 can obtain the filters 56 from a database 57, with which the application 30 can interact via the network 53. The database 57 can be updated with more filters 56, allowing the application 30 and present them to the user as the filters 56 become available.

Allowing a user to choose and selectively apply filters to selected portions of an ECG facilitates obtaining an ECG that includes discernible diagnostic information and can be used for patient diagnosis. FIGURE 5 is a flow diagram showing a method 60 for interactive processing of ECG data in accordance with one embodiment. Initially, an ECG 32 that is a result of electrocardiographic monitoring of a patient is obtained by the application 30 executed on the user device 31 (step 61). The ECG 32 can be obtained from an ECG monitor or from other sources, as described above, and can be obtained upon a completion of the monitoring, or continuously received in real-time as monitoring progresses. In a still further embodiment, both the ECG 32 and the digitized ECG signals corresponding to the ECG 32 can be obtained.

Optionally, if the application 30 identifies presence of noise, such as baseline wander, in the ECG received from a monitor or another source, the application 30 can automatically apply one or more filters to digitized signals corresponding to portions of the obtained ECG that has the noise, with the ECG 32 that is subsequently displayed to the user being generated based on the filtered digitized signals and signals for portions of the ECG that did not include the baseline wander, as further described above with reference to FIGURE 3 (step 62).

The ECG 32, after having been optionally automatically filtered, is displayed on a display screen of the user device 31 (step 63). If the ECG 32 is received over a period of time, such as when the ECG is a result of an ongoing electrocardiographic monitoring, portions of the ECG can be updated in real-time as they are being received, with the displayed ECG being updated as more results of the monitoring become available. If the ECG 32 is a result of an already completed monitoring, all portions of the ECG can be displayed at the same time.

A user selection 34 of a portion of the ECG is received, such as via the user touching the portion on the touch-screen display of the user device 31, entering the selection from a keyboard, or using a mouse (step 64). Digitized ECG signals corresponding to the selected portion 34 of the ECG are obtained by the application 30 (step 65). If the digitized signals for the ECG 32 were received with the ECG 32, the signals corresponding to the selection 34 can be identified among the received signals. If no digitized ECG signals have been received, the application 30 can reconstruct the digitized signals from the selection 34. Other ways to obtain the digitized signals are possible.

Optionally, the selection is zoomed and the zoomed selection 35 is displayed to the user by the application (step 66). A list, such as in the selection menus 36, 38, of a plurality of digital ECG filters for filtering the selection is displayed to the user, with the user being able to select one or more sets of the filters for filtering the selection (step 67). Optionally, a filter recommended for processing the selection 34 is determined and displayed to the user, as further described with reference to FIGURE 6 (step 68). A user selection of one or more sets of the filters is received by the application 30, with each of the filter sets including at least one of the filters displayed (step 69). The application 30 applies each of the sets of the selected filters to the digitized ECG signals for the selection (step 70), generates filtered ECG for the selection based on the digital signals filtered by each of the sets, and displays the filtered ECG for the selection on portions 37, 39 of the display screen of the user device 31 (step 71). The filtered ECGs can be displayed visually proximate to each other, allowing comparison of results of filtering side-by-side, and thus enabling the user to decide which of the results is more useful, whether one of the results satisfies the user's needs, or whether a still different set of filters needs to be applied. Optionally, upon receiving a user selection of one of the filtered ECGs for the selection, the application 30 can replace the selected portion 34 of the ECG 32 with the selected filtered ECG (step 72), ending the method 60.

Recommending an ECG filter to the user can save the user time and simplify ECG interpretation for the user. FIGURE 6 is a flow diagram showing a routine 80 for recommending an ECG filter to a user for use in the method 60 of FIGURE 5 in accordance with one embodiment. First, a frequency of a recursive noise present in the ECG selection is identified (step 81). Second, one or more digital filters are selected based on the noise frequency (step 82). For example, if the selection includes high-frequency recursive noise, a low-pass filter can be chosen for the recommendation. Lastly, the selected filter is recommended to a user, terminating the routine 80 (step 83).

While the invention has been particularly shown and described as referenced to the embodiments thereof, those skilled in the art will understand that the foregoing and other changes in form and detail may be made therein. The scope of the invention is defined by the claims.

## Claims

1. A system (40) for interactive processing of ECG data (10), comprising:
a display module configured to display (63) an electrocardiogram (ECG) (10);
a receipt module configured to receive (64) from a user a user selection of a portion (34) of the displayed ECG (10);
a signal module configured to obtain (65) digitized signals corresponding to the selection (34);
a list module configured to display (67) a list of digital filters (56) for filtering the selection (34);
a recommendation module configured to recommend (68) one of the filters (56) in the list to the user, comprising:
an identification module configured to identify (81) from the digitized signals a frequency of recursive noise in the selection (34);
an application (30) configured to select (82) one of the filters (56) as the recommended filter based on the frequency; and
the application (30) further configured to present (83) the selected filter (56) as the recommended filter;
a selection module configured to receive (69) a user selection from the user of one or more sets of the digital filters (56), each of the sets comprising one or more of the filters (56) from the list;
an application module configured to apply (70) the selected sets to the digitized signals for the-selection (34);
a generation module configured to generate (71) for each of the sets a filtered ECG (10) of the selection (34) based on the signals filtered by that set; and
a set module configured to display (71) the filtered selection ECG (10) for each of the sets.

2. A system (40) according to Claim 1, wherein the ECG (10) is based on electrocardiographic monitoring of a patient (43) performed by a long-term electrocardiographic monitor.

3. A system (40) according to Claim 1, wherein the filters (56) on the list comprise one or more of a low-pass filter, high-pass filter, notch filter, phase correction filter, and an adaptive filter.

4. A system (40) according to Claim 1, wherein the user selection of the filters (56) comprises at least two of the sets of the filters (56) and the filtered ECG (10) for the at least two sets are displayed visually proximate to each other.

5. A system (40) according to Claim 1, further comprising:
a filtered selection module configured to receive a user selection of the filtered selection ECG (10) for one of the sets; and
a replacement module configured to replace (72) the selection in the displayed ECG (10) with the filtered selection ECG (10).

6. A system (40) according to Claim 1, further comprising one or more of:
a signal receipt module configured to receive digitized signals for the ECG (10) comprising the digitized signals for the selection (34); and
an identification module configured to identify the digitized ECG (10) signals for the selection among the received digitized signals for the ECG (10).

7. A system (40) according to Claim 1, further comprising:
a noise module configured to obtain an ECG (10) corrupted by an ECG (10) noise;
a portion module configured to identify one or more portions of the corrupted ECG (10) comprising the noise;
an automation module configured to automatically apply one or more of the filters (56) to digitized signals for the portions of the corrupted ECG (10); and
an ECG generation module configured to generate the ECG (10) based on the automatically filtered digitized signals.

8. A method (60) for interactive processing of ECG data (10), comprising the steps of:
displaying (63) an electrocardiogram (ECG) (10);
receiving (64) from a user a user selection of a portion (34) of the displayed ECG (10);
obtaining (65) digitized signals corresponding to the selection (34);
displaying (67) a list of digital filters (56) for filtering the selection (34);
recommending (68) by a recommendation module one of the filters (56) in the list to the user, comprising the steps of:
identifying (81) by an identification module a frequency of recursive noise in the selection (34);
selecting (82) by an application (30) one of the filters (56) as the recommended filter based on the frequency; and
presenting (83) by an application (30) the selected filter as the recommended filter;
receiving a user selection from the user of one or more sets of the digital filters (56), each of the sets comprising one or more of the filters (56) from the list;
applying (70) the selected sets to the digitized signals for the-selection (34);
generating (71) for each of the sets a filtered ECG (10) of the selection (34) based on the signals filtered by that set; and
displaying (72) the filtered selection ECG (10) for each of the sets.

9. A method (60) according to Claim 8, wherein the ECG (10) is based on electrocardiographic monitoring of a patient (43) performed by a long-term electrocardiographic monitor.

10. A method (60) according to Claim 9, wherein the monitor is located along the patient's (43) sternum (42) when performing the monitoring.

11. A method (60) according to Claim 8, wherein the filters (56) on the list comprise one or more of a low-pass filter, high-pass filter, notch filter, phase correction filter, and an adaptive filter.

12. A method (60) according to Claim 8, wherein the user selection of the filters (56) comprises at least two of the sets of the filters (56) and the filtered ECG (10) for the at least two sets are displayed visually proximate to each other.

13. A method (60) according to Claim 8, further comprising the steps of:
receiving a user selection of the filtered selection s for one of the sets; and
replacing (72) the selection in the displayed ECG (10) with the filtered selection ECG (10).

14. A method (60) according to Claim 8, further comprising the steps of:
receiving digitized signals for the ECG (10) comprising the digitized signals for the selection; and
identifying the digitized ECG (10) signals for the selection (34) among the received digitized signals for the ECG (10).

15. A method (60) according to Claim 8, further comprising the steps of:
obtaining an ECG (10) corrupted by an ECG (10) noise;
identifying one or more portions of the corrupted ECG (10) comprising the noise;
automatically applying one or more of the filters (56) to digitized signals for the portions of the corrupted ECG (10); and
generating the ECG (10) based on the automatically filtered digitized signals.

## Patentansprüche

1. System (40) zur interaktiven Verarbeitung von EKG-Daten (10), umfassend:
ein Anzeigemodul, das dazu ausgelegt ist, ein Elektrokardiogramm (EKG) (10) anzuzeigen (63);
ein Empfangsmodul, das dazu ausgelegt ist, von einem Benutzer eine Benutzerauswahl eines Abschnitts (34) des angezeigten EKGs (10) zu empfangen (64);
ein Signalmodul, das dazu ausgelegt ist, digitalisierte Signale zu erhalten (65), die der Auswahl (34) entsprechen;
ein Listenmodul, das dazu ausgelegt ist, eine Liste digitaler Filter (56) zum Filtern der Auswahl (34) anzuzeigen (67);
ein Empfehlungsmodul, das dazu ausgelegt ist, dem Benutzer einen der Filter (56) in der Liste zu empfehlen (68), umfassend:
ein Identifizierungsmodul, das dazu ausgelegt ist, aus den digitalisierten Signalen eine Frequenz des rekursiven Rauschens in der Auswahl (34) zu identifizieren (81);
eine Anwendung (30), die dazu ausgelegt ist, einen der Filter (56) als den empfohlenen Filter basierend auf der Frequenz auszuwählen (82); und
die Anwendung (30) ferner dazu ausgelegt ist, den ausgewählten Filter (56) als den empfohlenen Filter darzustellen (83),
ein Auswahlmodul, das dazu ausgelegt ist, eine Benutzerauswahl von dem Benutzer aus einem oder mehreren Sätzen der digitalen Filter (56) zu empfangen (69), wobei jeder der Sätze einen oder mehrere der Filter (56) von der Liste umfasst;
ein Anwendungsmodul, das dazu ausgelegt ist, die ausgewählten Sätze auf die digitalisierten Signale für die Auswahl (34) anzuwenden (70);
ein Erzeugungsmodul, das dazu ausgelegt ist, für jeden der Sätze ein gefiltertes EKG (10) der Auswahl (34) basierend auf den von dem Satz gefilterten Signalen zu erzeugen; und
ein Satzmodul, das dazu ausgelegt ist, die gefilterte Auswahl des EKGs (10) für jeden der Sätze anzuzeigen (71).

2. System (40) nach Anspruch 1, wobei das EKG (10) auf der elektrokardiografischen Überwachung eines Patienten (43) basiert, die von einem Langzeitelektrokardiografiemonitor durchgeführt wird.

3. System (40) nach Anspruch 1, wobei die Filter (56) auf der Liste einen oder mehrere von einem Tiefpassfilter, einem Hochpassfilter, einem Kerbfilter, einem Phasenkorrekturfilter und einem adaptiven Filter umfassen.

4. System (40) nach Anspruch 1, wobei die Benutzerauswahl der Filter (56) mindestens zwei der Sätze der Filter (56) umfasst und die gefilterten EKGs (10) für die mindestens zwei Sätze visuell nahe beieinander angezeigt werden.

5. System (40) nach Anspruch 1, ferner umfassend:
ein Modul für die gefilterte Auswahl, das dazu ausgelegt ist, eine Benutzerauswahl des gefilterten EKGs (10) der Auswahl für einen der Sätze zu empfangen; und
ein Ersatzmodul, das dazu ausgelegt ist, die Auswahl in dem angezeigten EKG (10) durch das gefilterte EKG (10) der Auswahl zu ersetzen (72).

6. System (40) nach Anspruch 1, ferner umfassend eins oder mehrere von:
einem Signalempfangsmodul, das dazu ausgelegt ist, digitalisierte Signale für das EKG (10) zu empfangen, die die digitalisierten Signale für die Auswahl (34) umfassen; und
ein Identifizierungsmodul, das dazu ausgelegt ist, die digitalisierten EKG (10)-Signale für die Auswahl aus den empfangenen digitalisierten Signalen für das EKG (10) zu identifizieren.

7. System (40) nach Anspruch 1, ferner umfassend:
ein Rauschmodul, das dazu ausgelegt ist, ein EKG (10) zu erhalten, das durch ein EKG (10)-Rauschen beschädigt ist;
ein Abschnittsmodul, das dazu ausgelegt ist, einen oder mehrere Abschnitte des beschädigten EKGs (10), die das Rauschen umfassen, zu identifizieren;
ein Automatisierungsmodul, das dazu ausgelegt ist, automatisch einen oder mehrere der Filter (56) auf die digitalen Signale für die Abschnitte des beschädigten EKGs (10) anzuwenden; und
ein EKG-Erzeugungsmodul, das dazu ausgelegt ist, das EKG (10) basierend auf den automatisch gefilterten digitalisierten Signalen zu erzeugen.

8. Verfahren (60) zur interaktiven Verarbeitung von EKG-Daten (10), umfassend die Schritte:
Anzeigen (63) eines Elektrokardiogramms (EKG) (10);
Empfangen (64) einer Benutzerauswahl eines Abschnitts (34) des angezeigten EKGs (10) von einem Benutzer;
Erhalten (65) digitalisierter Signale, die der Auswahl (34) entsprechen;
Anzeigen (67) einer Liste digitaler Filter (56) zum Filtern der Auswahl (34);
dem Benutzer Empfehlen (68) von einem der Filter (56) in
der Liste durch das Empfehlungsmodul, umfassend die Schritte:
Identifizieren (81) einer Frequenz des rekursiven Rauschens in der Auswahl (34) durch ein Identifizierungsmodul;
Auswählen (82) von einem der Filter (56) als den empfohlenen Filter basierend auf der Frequenz durch eine Anwendung (3); und
Darstellen (83) des ausgewählten Filters als den empfohlenen Filter durch eine Anwendung (30);
Empfangen einer Benutzerauswahl aus einem oder mehreren Sätzen der digitalen Filter (56) von dem Benutzer, wobei jeder der Sätze einen oder mehrere der Filter (56) von der Liste umfasst;
Anwenden (70) der ausgewählten Sätze auf die digitalisierten Signale für die Auswahl (34);
Erzeugen (71) für jeden der Sätze eines gefilterten EKGs (10) der Auswahl (34) basierend auf den von dem Satz gefilterten Signalen; und
Anzeigen (72) der gefilterten Auswahl des EKGs (10) für jeden der Sätze.

9. Verfahren (60) nach Anspruch 8, wobei das EKG (10) auf der elektrokardiografischen Überwachung eines Patienten (43) basiert, die von einem Langzeitelektrokardiografiemonitor durchgeführt wird.

10. Verfahren (60) nach Anspruch 9, wobei sich der Monitor bei Durchführung der Überwachung entlang des Brustbeins (42) des Patienten (43) befindet.

11. Verfahren (60) nach Anspruch 8, wobei die Filter (56) auf der Liste einen oder mehrere von einem Tiefpassfilter, einem Hochpassfilter, einem Kerbfilter, einem Phasenkorrekturfilter und einem adaptiven Filter umfassen.

12. Verfahren (60) nach Anspruch 8, wobei die Benutzerauswahl der Filter (56) mindestens zwei der Sätze der Filter (56) umfasst und die gefilterten EKGs (10) für die mindestens zwei Sätze visuell nahe beieinander angezeigt werden.

13. Verfahren (60) nach Anspruch 8, ferner umfassend die Schritte:
Empfangen einer Benutzerauswahl der gefilterten Auswahl für einen der Sätze; und
Ersetzen (72) der Auswahl in dem angezeigten EKG (10) durch das gefilterte EKG (10) der Auswahl.

14. Verfahren (60) nach Anspruch 8, ferner umfassend die Schritte:
Empfangen digitalisierter Signale für das EKG (10), umfassend die digitalisierten Signale für die Auswahl; und
Identifizieren der digitalisierten EKG (10)-Signale für die Auswahl (34) aus den empfangenen digitalisierten Signalen für das EKG (10).

15. Verfahren (60) nach Anspruch 8, ferner umfassend die Schritte:
Erhalten eines EKGs (10), das durch ein EKG (10)-Rauschen beschädigt ist;
Identifizieren von einem oder mehreren Abschnitten des beschädigten EKGs (10), die das Rauschen umfassen;
automatisches Anwenden von einem oder mehreren der Filter (56) auf die digitalisierten Signale für die Abschnitte des beschädigten EKGs (10); und
Erzeugen des EKGs (10) basierend auf den automatisch gefilterten digitalisierten Signalen.

## Revendications

1. Système (40) de traitement interactif de données ECG (10) comprenant :
un module d'affichage configuré pour afficher (63) un électrocardiogramme (ECG) (10) ;
un module de réception configuré pour recevoir (64), en provenance d'un utilisateur, une sélection, par l'utilisateur, d'une partie (34) de l'ECG (10) affiché ;
un module de signal configuré pour obtenir (65) des signaux numérisés correspondant à la sélection (34) ;
un module de liste configuré pour afficher (67) une liste de filtres numériques (56) pour filtrer la sélection (34) ;
un module de recommandation configuré pour recommander (68), à l'utilisateur, l'un des filtres (56) de la liste, comprenant :
un module d'identification configuré pour identifier (81), à partir des signaux numérisés, une fréquence de bruit récurrent dans la sélection (34) ;
une application (30) configurée pour sélectionner (82) l'un des filtres (56) en tant que filtre recommandé, sur la base de la fréquence ; et
l'application (30) étant en outre configurée pour présenter (83) le filtre sélectionné (56) en tant que filtre recommander ;
un module de sélection configuré pour recevoir (69), en provenance de l'utilisateur, une sélection, par l'utilisateur, d'un ou plusieurs ensembles des filtres numériques (56), chacun des ensembles comprenant un ou plusieurs filtres (56) de la liste ;
un module d'application configuré pour appliquer (70) les ensembles sélectionnés aux signaux numérisés pour la sélection (34) ;
un module de génération configuré pour générer (71), pour chacun des ensembles, un ECG (10) filtré de la sélection (34) sur la base des signaux filtrés par cet ensemble ; et
un module d'ensemble configuré pour afficher (71) l'ECG (10) de sélection filtré pour chacun des ensembles.

2. Système (40) selon la revendication 1, dans lequel l'ECG (10) est basé sur une surveillance électrocardiographique d'un patient (43) réalisée par un moniteur électrocardiographique à long terme.

3. Système (40) selon la revendication 1, dans lequel les filtres (56) de la liste comprennent un ou plusieurs parmi un filtre passe-bas, un filtre passe-haut, un filtre coupe-bande, un filtre à correction de phase et un filtre adaptatif.

4. Système (40) selon la revendication 1, dans lequel la sélection, par l'utilisateur, des filtres (56) comprend au moins deux des ensembles des filtres (56) et les ECG (10) filtrés pour les au moins deux ensembles sont affichés à proximité visuelle l'un de l'autre.

5. Système (40) selon la revendication 1, comprenant en outre :
un module de sélection filtrée configuré pour recevoir une sélection, par l'utilisateur, de l'ECG (10) de sélection filtré pour l'un des ensembles ; et
un module de remplacement configuré pour remplacer (72) la sélection dans l'ECG (10) affiché par l'ECG (10) de sélection filtré.

6. Système (40) selon la revendication 1 comprenant en outre un ou plusieurs parmi :
un module de réception de signaux configuré pour recevoir des signaux numérisés pour l'ECG (10) comprenant les signaux numérisés pour la sélection (34) ; et
un module d'identification configuré pour identifier les signaux d'ECG (10) numérisés pour la sélection parmi les signaux numérisés reçus pour l'ECG (10).

7. Système (40) selon la revendication 1, comprenant en outre :
un module de bruit configuré pour obtenir un ECG (10) corrompu par un bruit d'ECG (10) ;
un module de partie configuré pour identifier une ou plusieurs parties de l'ECG (10) corrompu comprenant le bruit ;
un module d'automatisation configuré pour appliquer automatiquement un ou plusieurs des filtres (56) aux signaux numérisés pour les parties de l'ECG (10) corrompu ; et
un module de génération d'ECG configuré pour générer l'ECG (10) sur la base des signaux numérisés filtrés automatiquement.

8. Procédé (60) de traitement interactif de données ECG (10), comprenant les étapes :
afficher (63) un électrocardiogramme (ECG) (10) ;
recevoir (64), en provenance d'un utilisateur, une sélection, par l'utilisateur, d'une partie (34) de l'ECG (10) affiché ;
obtenir (65) des signaux numérisés correspondant à la sélection (34) ;
afficher (67) une liste de filtres numériques (56) pour filtrer la sélection (34) ;
recommander (68), par un module de recommandation, l'un des filtres (56) dans la liste de l'utilisateur, comprenant les étapes :
identifier (81), par un module d'identification, une fréquence de bruit récurrent dans la sélection (34) ;
sélectionner (82), par une application (30), l'un des filtres (56) en tant que filtre recommandé, sur la base de la fréquence ; et
présenter (83), par une application (30), le filtre sélectionné en tant que filtre recommandé ;
recevoir (69), en provenance de l'utilisateur, une sélection, par l'utilisateur, d'un ou plusieurs ensembles des filtres numériques (56), chacun des ensembles comprenant un ou plusieurs des filtres (56) de la liste ;
appliquer (70) les ensembles sélectionnés aux signaux numérisés pour la sélection (34) ;
générer (71), pour chacun des ensembles, un ECG (10) filtré de la sélection (34) sur la base des signaux filtrés par cet ensemble ; et
afficher (72) l'ECG (10) de sélection filtré pour chacun des ensembles.

9. Procédé (60) selon la revendication 8, dans lequel l'ECG (10) est basé sur une surveillance électrocardiographique d'un patient (43) réalisée par un moniteur électrocardiographique à long terme.

10. Procédé (60) selon la revendication 9, dans lequel le moniteur est situé le long du sternum (42) du patient (43) lors de la réalisation de la surveillance.

11. Procédé (60) selon la revendication 8, dans lequel les filtres (56) de la liste comprennent un ou plusieurs parmi un filtre passe-bas, un filtre passe-haut, un filtre coupe-bande, un filtre à correction de phase et un filtre adaptatif.

12. Procédé (60) selon la revendication 8, dans lequel la sélection, par l'utilisateur, des filtres (56) comprend au moins deux des ensembles des filtres (56) et les ECG (10) filtrés pour les au moins deux ensembles sont affichés à proximité visuelle l'un de l'autre.

13. Procédé (60) selon la revendication 8,comprenant en outre les étapes :
recevoir une sélection, par l'utilisateur, de l'ECG (10) de sélection filtré pour l'un des ensembles ; et
remplacer (72) la sélection dans l'ECG (10) affiché par l'ECG (10) de sélection filtré.

14. Procédé (60) selon la revendication 8, comprenant en outre les étapes :
recevoir des signaux numériques pour l'ECG (10) comprenant les signaux numérisés pour la sélection ; et
identifier les signaux d'ECG (10) numérisés pour la sélection (34) parmi les signaux numérisés reçus pour l'ECG (10) .

15. Procédé (60) selon la revendication 8, comprenant en outre les étapes :
obtenir un ECG (10) corrompu par un bruit d'ECG (10) ;
identifier une ou plusieurs parties de l'ECG (10) corrompu comprenant le bruit ;
appliquer automatiquement un ou plusieurs des filtres (56) aux signaux numérisés pour les parties de l'ECG (10) corrompu ; et
générer l'ECG (10) sur la base des signaux numérisés filtrés automatiquement.
